# EUROPÄISCHE PATENTSCHRIFT

(11) **EP 0 065 661 B1**
(45) Veröffentlichungstag und Bekanntmachung des Hinweises auf die Patenterteilung: **05.06.1985**
(21) Anmeldenummer: 82103590.4
(22) Anmeldetag: 28.04.1982
(51) Int. Cl.: C08F 2/26, C08F 4/04, C07C 107/02

(54) **Oberflächenaktive Azoverbindungen und ihre Verwendung**
Surface-active azo compounds and their use
Composés azo tensio-actives et leur usage

(30) Priorität: 09.05.1981 DE 3118373
(43) Veröffentlichungstag der Anmeldung: 01.12.1982
(73) Patentinhaber: BAYER AG, 51368 Leverkusen (DE)
(72) Erfinder: Schmidt, Adolf, Dr., D-5000 Köln 80 (DE); Roos, Ernst, Dr., D-5068 Odenthal (DE)

## Beschreibung

Die Erfindung betrifft Azo- und Harnstoff- und Carboxylat- bzw. Sulfonatgruppen enthaltende, wasserlösliche, oberflächenaktive Substanzen, die als emulgierend wirkende Initiatoren zur Herstellung von elektrolytarmen Dispersionen mit einer geringen Tendenz zur Schaumbildung auf Basis olefinisch ungesättigter Monomerer eingesetzt werden können.

Es ist bekannt, Azo-di-isobuttersäureamidin und davon abgeleitete Derivate, z. B. N-Alkylierungs- und N-Oxalkyfierungsprodukte in Form der Salze oder freien Basen als wasserlösliche Initiatoren bei der Emulsionspolymerisation olefinisch ungesättigter Monomerer einzusetzen (vgl. US-PS 2 599 300, 2 810 702, DE-OS 2 841 045).

Es ist weiter bekannt, α,α'-Azo-(α-methyl-γ·sulfo)-buttersäuredinitriI der Formel 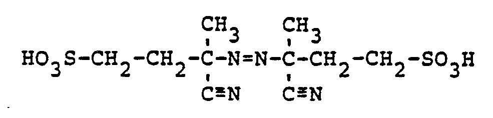 (vgl. DE-AS 1111 395 = US-PS 3161 630) oder α,α'-Azo(α-methyl-y-carboxyl)-buttersäuredinitril der Formel 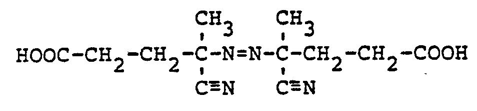 (vgl. US-PS 2 520 338), vorzugsweise in Form ihrer Salze als wasserlösliche Initiatoren für die Polymerisation olefinisch ungesättigter Monomerer einzusetzen. Für die Emulsionspolymerisation olefinisch ungesättigter Monomerer ohne Zusatz üblicher Emulgatoren zur Herstellung stabiler Dispersionen sind sie jedoch nicht geeignet, wie Vergleichsversuche in der vorliegenden Anmeldung zeigen.

Es ist schließlich auch bekannt (vgl. DE-OS 2 242 520 = GB-PS 1 402 060) isocyanatgruppen enthaltende Azoverbindungen der allgemeinen Formel 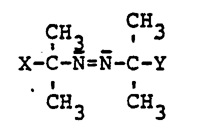 herzustellen, worin
X und Y gleich oder verschieden, beispielsweise die nachstehenden Reste bedeuten : 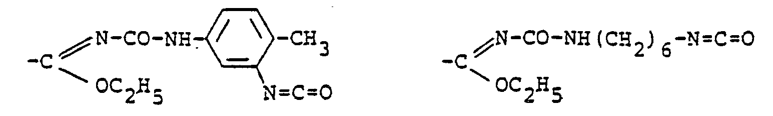 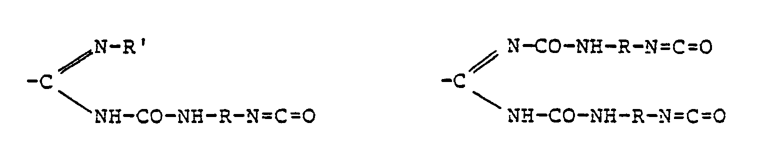
R' Alkyl, Cycloalkyl, Aryl, Aralkyl ;
R Alkylen, Cycloalkylen, Arylen, die Alkylenreste können durch einen cycloaliphatischen oder aromatischen Rest unterbrochen sein, die mehrkernigen Cycloalkylen oder mehrkernigen Arylenreste können durch einen aliphatischen Rest unterbrochen sein.

Die vorgenannten Verbindungen werden als Radikalbildner bei der Herstellung von Isocyanatgruppen enthaltenden Telomerisaten auf Basis olefinisch ungesättigter Monomerer verwendet. Gemäß vorliegender Erfindung dienen sie als Ausgangsstoffe bei der Herstellung der Azogruppen enthaltenden Emulgatoren.

Die in Gegenwart der Azoinitiatoren der vorgenannten Schriften und Emulgatoren herstellbaren Dispersionen neigen zur Schaumbildung. Aufgabe der vorliegenden Erfindung war daher das Bereitstellen eines Initiator-Emulgator-Systems, das die Herstellung von Dispersionen mit möglichst geringer Tendenz zur Schaumbildung erlaubt. Gleichzeitig sollten die Dispersionen einen möglichst geringen Elektrolytgehalt aufweisen.

Die Aufgabe wurde dadurch gelöst, daß neue oberflächenaktive Azoverbindungen mit eingebauten Harnstoff- und Carboxylat- bzw. Sulfonatgruppen zur Verfügung gestellt wurden, die gleichzeitig emulgierende und aktivierende Eigenschaften besitzen. Diese neuen Substanzen wirken zunächst als Emulgatoren, zerfallen während der Polymerisation, wobei die Katalysatorbruchstücke mit den Carboxylat- bzw. Sulfonatgruppen in das Polymerisat eingebaut werden und die Latexteilchen vor Koagulation schützen.

Gegenstand der Erfindung sind somit oberflächenaktive Azoverbindungen der allgemeinen Formel (A) 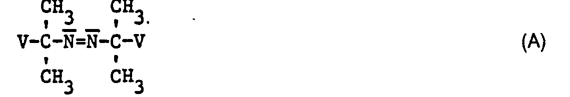 worin V die Bedeutung 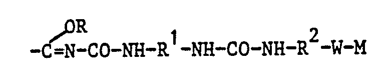 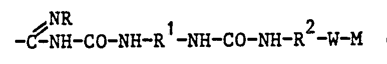 oder 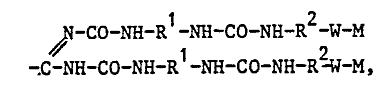 hat, wobei
; R = Alkyl mit 1-4 C-Atomen,
R¹ = den um die Isocyanatgruppen verminderten Rest eines aliphatischen, aromatischen oder cycloaliphatischen Diisocyanats,
R² = Alkylen mit 5-12 C-Atomen oder p-Phenylen
W = ―COO⁽⁻⁾, ―SO₃⁽⁻⁾ und
M = Na⁽⁺⁾, K⁽⁺⁾, NH₄⁽⁺⁾ bedeuten.
Vorzugsweise bedeuten in vorstehender Formel 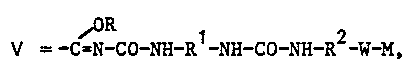
R = C₂H₅ ^{.}
R¹ = den um die Isocyanatgruppen verminderten Rest des 2,4- oder 2,6-Toluylendiisocyanats bzw. von deren Mischungen ; des 4.4'-Diisocyanatodiphenylmethans. des Hexamethylendüsocyanats, des 2,4,4-Trimethyl-1,6-diisocyanatohexans, des 1,12-Dodecamethylendiisocyanats, des Isophoron-diisocyanats,
R² = Alkylen mit 5-10 C-Atomen, Phenylen
W = ―COO⁽⁻⁾. -S0₃⁹ und
M = Na⁽⁺⁾, K⁽⁺⁾.

Die für die Herstellung der Azoverbindungen (A) notwendigen Isocyanate (B) sind bekannt (vgl. DE-OS 2 242 520 = GB-PS 1 402 060) bzw. können gemäß den zitierten Schriften aus den entsprechenden Azoiminoethern bzw. Azoamidinen und den bekannten aliphatischen, cycloaliphatischen oder aromatischen Diisocyanaten hergestellt werden.

Die Herstellung der Azoverbindungen (A) kann durch Umsetzen der Isocyanate der allgemeinen Formel (B) 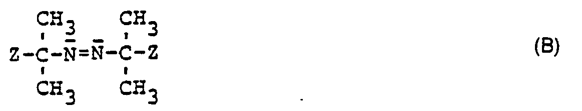 worin
Z die Bedeutung 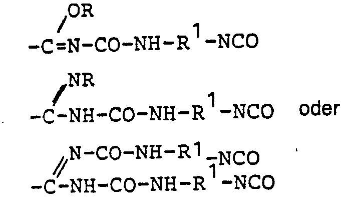 hat, und
R und R¹ die für Formel (A) angegebene Bedeutung haben, mit im wesentlichen äquivalenten Mengen eines Alkalimetall- oder Ammoniumsalzes einer w-Aminoalkylcarbonsäure bzw. w-Aminoalkylsulfonsäure mit einer primären Aminogruppe und mit 5-12 C-Atomen, vorzugsweise 5-10 C-Atomen in der Alkylgruppe oder mit aromatischen Aminosulfonsäuren wie p-aminobenzolsulfonsäure, erfolgen (1 NH₂-Gruppe = 1 Isocyanatgruppe), und zwar in organischer oder organisch-wäßriger oder wäßriger Lösung oder Suspension. Im Falle der Anwesenheit von Wasser muß gewährleistet sein, daß das Isocyanat mit den primären Aminogruppen der Aminoalkylsäure schneller reagiert als mit Wasser. Die Umsetzung kann bei 0 °C-35 °C, vorzugsweise 5 °C-25 °C, durchgeführt werden.

Als organisches Lösungsmittel kann Tetrahydrofuran, Dimethylformamid, Aceton, Methylethylketon, Dioxan, Acetonitril, Dimethylsulfoxid oder deren Gemische eingesetzt werden.

Die Aufarbeitung des Reaktionsgemisches kann durch schonendes Eindampfen bis zur Trockne erfolgen, Der Rückstand wird dann mit Wasser, methanol oder Ethanol versetzt, wobei die Carboxylat- bzw. Sulfonatgruppen enthaltenden Azoverbindungen in Lösung gehen. Eine andere Möglichkeit der Aufarbeitung besteht in der Extraktion des wäßrigen Reaktionsgemisches mit geeigneten, mit Wasser nicht mischbaren Lösungsmitteln wie niedrig-siedenden Ethern.

Gegenstand der Erfindung ist auch die Verwendung der Azoverbindungen der Formel (A) bei der Emulsionspolymerisation eines oder mehrerer olefinisch ungesättigter Monomerer zur Herstellung von elektrolytarmen Dispersionen mit geringer Tendenz zur Schaumbildung. Die Verbindungen der Formel (A) werden in Mengen von 1,5-5 Gew.-%, vorzugsweise 2-3 Gew.-%. bezogen auf Monomere, eingesetzt.

Die erfindungsgemäßen Azoverbindungen können bei der Polymerisation wie übliche Emulgatoren gehandhabt werden, d. h. sie können entweder vollständig vorgelegt werden oder sie können im Rahmen eines Zulaufverfahrens zum Teil vorgelegt und zum Teil während der Emulsionspolymerisation nachdosiert werden.

Entsprechend der Zerfallskinetik der Azoverbindungen wird die Polymerisation bei Temperaturen zwischen 35 °C bis 90 °C durchgeführt. Der bevorzugte Temperaturbereich beträgt 45 °C bis 75 °C.

An polymerisierbaren Monomeren kommen alle üblicherweise mit Azodiisobuttersäure-Nitril polymerisierbaren, olefinisch ungesättigten Monomeren in Betracht, beispielsweise Styrol, L-Methylstyrol, Butadien, Acrylsäureester mit 1-8 C-Atomen in der Alkoholkomponente, Methacrylsäureester mit 1-8 C-Atomen in der Alkoholkomponente, Acrylnitril, Methacrylnitril, Vinylchlorid, Vinylacetat, Ethylen, Chloropren usw.

Neben den genannten Monomeren können zusätzlich, in gegingerem Anteil, wasserlösliche Verbindungen wie Methacrylsäure, Acrylsäure, Maleinsäurehalbester, Itaconsäure und Itaconsäurehalbester, Acrylamid, Methacrylamid usw. einpolymerisiert werden. Ferner können noch funktionelle Gruppen, z. B. OH- oder Epoxigruppen tragenden Comonomere mitverwendet werden, wie β-Hydroxyethyl(meth)acrylat, β-Hydroxypropyl(meth)acrylat, Glycidyl(meth)acrylat und N-Methylol- bzw. N-Methylolalkylether-des (Meth)Acrylsäureamids.

Prinzipiell ist es natürlich möglich, die erfindungsgemäßen oberflächenaktiven Azoverbindungen in Kombination mit üblichen anionischen oder nichtionischen Emulgatoren einzusetzen.

Die vorteilhaften und überraschenden Eigenschaften der erfindungsgemäßen oberflächenaktiven Azoverbindungen gehen aus den Beispielen und Vergleichsversuchen dieser Anmeldung hervor.

Die in den Beispielen und Vergleichsversuchen angegebenen Prozente und Teile beziehen sich auf das Gewicht, sofern nicht anders vermerkt.

### Beispiel 1 (Azoemulgator 1)

Zu einer Lösung von
43 g (0,2 Mol) w-Aminoundecansäure
11,22g (0,2 Mol) Kaliumhydroxid
200 g Wasser
200 g Tetrahydrofuran (= THF)
   wird eine Suspension von
60,4 g (0,1 Mol) der Verbindung : 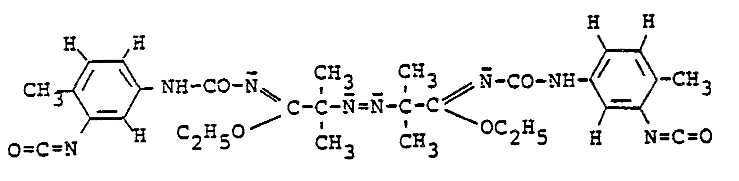 welche in 400 g THF gelöst bzw. suspendiert ist, hinzugetropft, wobei die Innentemperatur auf 25 °C gehalten wird. Nach ca. 24 stündigem Nachrühren bei Zimmertemperatur wird das Reaktionsgemisch zur Entfernung des Tetrahydrofurans im Vakuum zur Trockne eingeengt.

Der trockene Rückstand wird dann zur Auflösung des bei der Reaktion entstandenen Azoemulgators 3 Stunden bei 30 °C mit 750 ml Wasser intensiv verrührt. Nach Abfiltrieren nicht gelöster Anteile wird das weißlich getrübte Filtrat mit Wasser auf einen Feststoffgehalt von 10 % eingestellt. Diese 10 %ige Lösung des Azoemulgators wird für die in Beispiel 2 geschilderten Emulsionspolymerisationsversuche verwendet.

Idealisierte Struktur des Azoemulgators :

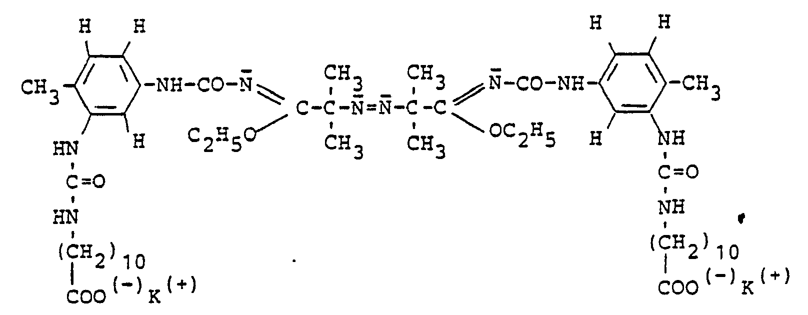

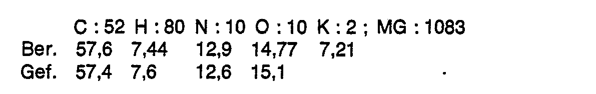

### Beispiel 2 (Polymerisation mit dem erfindungsgemäßen Emulgator)

Serienpolymerisationsversuche wurden in 500 ml fassenden verkorkten Glasflaschen mit zusätzlichem Schraubkappenverschluß (vgl. Houben-Weyl, Methoden der Organischen Chemie, Band 14/1, Seite 147, (1961)) unter Ausschluß von Luftsauerstoff durchgeführt. Die zum Splitterschutz in Edelstahlkörbe eingesetzten Glasflaschen rotierten bei einer Drehzahl von 25 Umdrehungen pro Minute. Die Temperatur des Wasserbades wurde konstant gehalten. Einzelheiten der Polymerisation und Eigenschaften der erhaltenen Dispersionen gehen aus Tabelle I hervor.

In allen Fällen entstehen Latices mit nahezu theoretischem Feststoffgehalt (d. h. quantitativem Monomerenumsatz). Der Koagulatanteil liegt niedrig wie bei Ansätzen mit gut wirksamen anionischen Tensiden.

Bei allen Latices (mit Ausnahme des Ansatzes Id) bricht nach dem Schütteln der Schaum schnell zusammen. Die Latexteilchendurchmesser sind mit 90-125 nm klein und liegen in derselben Größenordnung wie jene, welche mit anionischen Tensiden wie beispielsweise Natriumlaurat oder Natriumlaurylsulfat erhalten werden.

Das verwendete Leitfähigkeitsmeßgerät. zeigte die Leitfähigkeit von Kaliumchloridlösungen bei Zimmertemperatur wie folgt an :

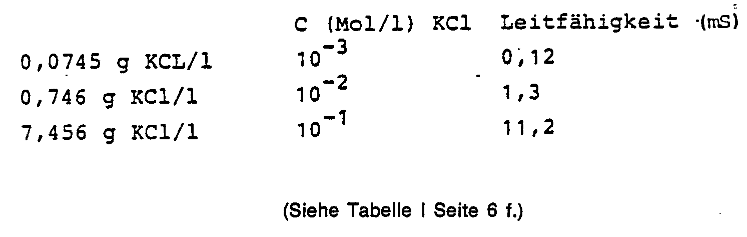

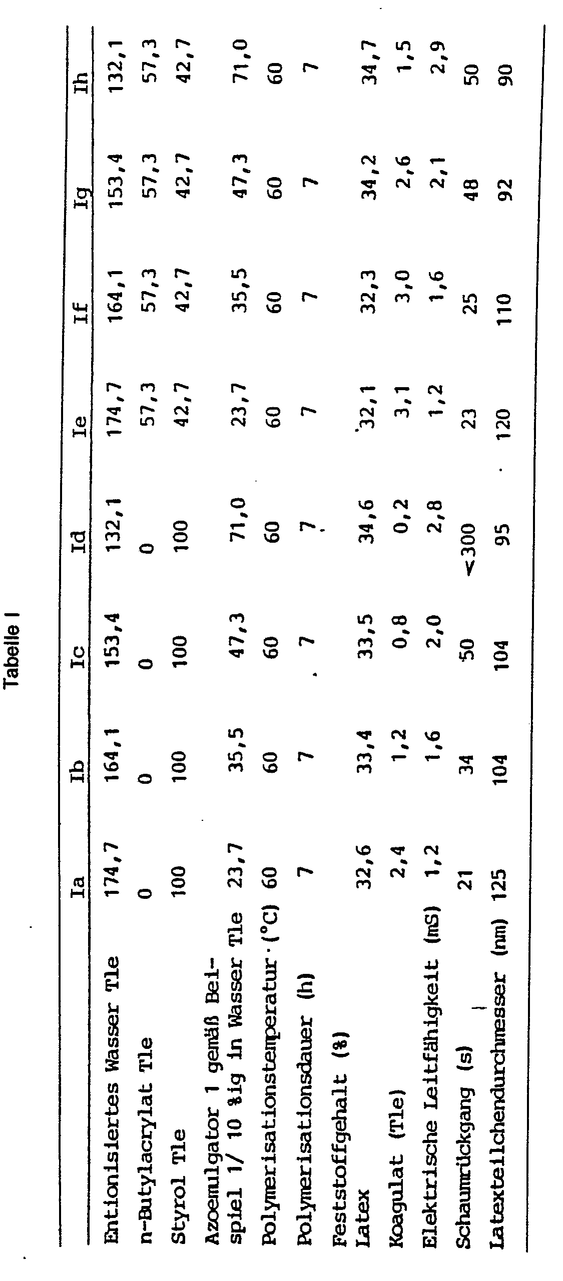

### Beispiel 3 (Azoemulgator 2 gemäß Erfindung)

Zu einer Lösung von
39,3 g Aminohexansäure (0,3 Mol)
40 g entionisiertes Wasser
33,6 g 50 %iger Kalilauge (0,3 Mol KOH)
40 g Tetrahydrofuran
   wird eine Suspension von
90,6 g (0,15 Mol) der Verbindung : 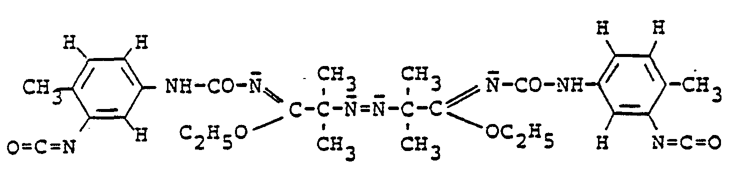 in 550 g Aceton bei + 10 °C in ca. 20 Minuten zugetropft. Nach 3-stündigem Rühren bei Zimmertemperatur wird das Reaktionsgemisch zur Entfernung des Acetons im Vakuum zur Trockne eingeengt. Der trockene Rückstand wird zur Auflösung des bei der Reaktion entstandenen Azoemulgators bei 25-30 °C 4 Stunden lang mit 800 ml Wasser verrührt, wobei im Reaktionsgemisch enthaltene Brocken mechanisch zerkleinert werden müssen. Nach Abfiltrieren nicht gelöster Bestandteile wird das Filtrat mit Wasser auf einen Feststoffgehalt von 10 % eingestellt. Diese 10 %ige Lösung wird für die in Beispiel 2 geschilderten Emulsionspolymerisationsversuche verwendet.

Idealisierte Struktur des Azoemulgators 2

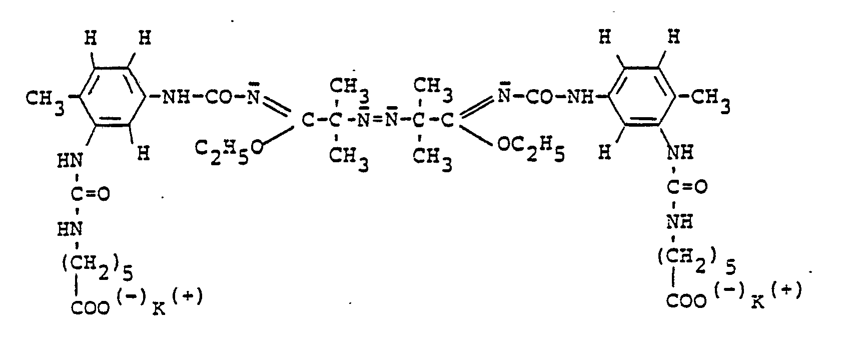

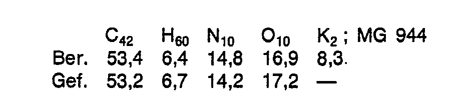

Im ¹H NMR-Spektrum, gemessen in D₂0 erscheinen stark verbreiterte Signale für aromatische Wasserstoffe bei 7,0 bis 7,4 ppm, die 0-18CH₂-Gruppe mit Zentrum um 4,1 ppm, die zum acylierten Stickstoff a-ständige CH₂⁶⁻Gruppe um 3 ppm, die am Aromaten stehende CH₃⁶⁻Gruppe um 2 ppm, die geminalen CH₃-Gruppen am quartären C um 1,3 ppm und die restlichen CH₂-Gruppen und die CH₃-Gruppen aus dem Ethoxyrest zwischen 1,1 bis 1,6 ppm.

Der Azoemulgator wird analog der in Beispiel 2 geschilderten Verfahrensweise für die Emulsionspolymerisation eingesetzt. Einzelheiten und Eigenschaften der erhaltenen Dispersion sind aus Tabelle II ersichtlich.
(Siehe Tabelle II Seite 8 f.)

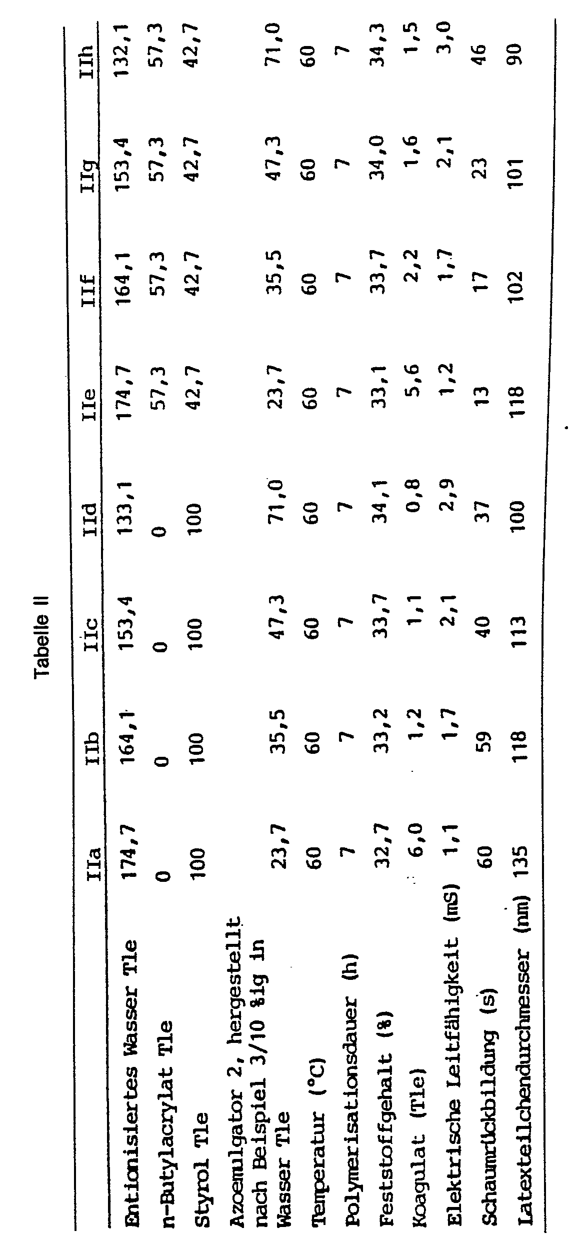

### Vergleichsversuche (Tabelle III)

Ein den bisherigen Versuchen entsprechendes n-Butyl-acrylat-Styrol Monomerengemisch wird in Gegenwart von Azodiisobuttersäureamidin als Initiator und in Gegenwart von
a) dem Di-Kaliumsalz einer handelsüblichen C36-Dimersäure, erhältlich durch Polymerisation ungesättigter C₁₈-Fettsäuren, mit der Struktur einer Dicarboxylsäure mit langer Kette und mit 2 oder mehr Alkylseitenketten, Säurezahl 191-197, Verseifungszahl 193-200, Viskosität bei 25 °C von 5200 m.Pa.s
b) dem Kaliumsalz der ω-Aminohexansäure,
c) dem Kaliumsalz der ω-Aminoundecansäure

als Emulgator analog Beispiel 2 polymerisiert.

Einzelheiten und Eigenschaften der Dispersionen sind aus Tabelle III erhältlich.
(Siehe Tabelle III Seite 10 ff.)

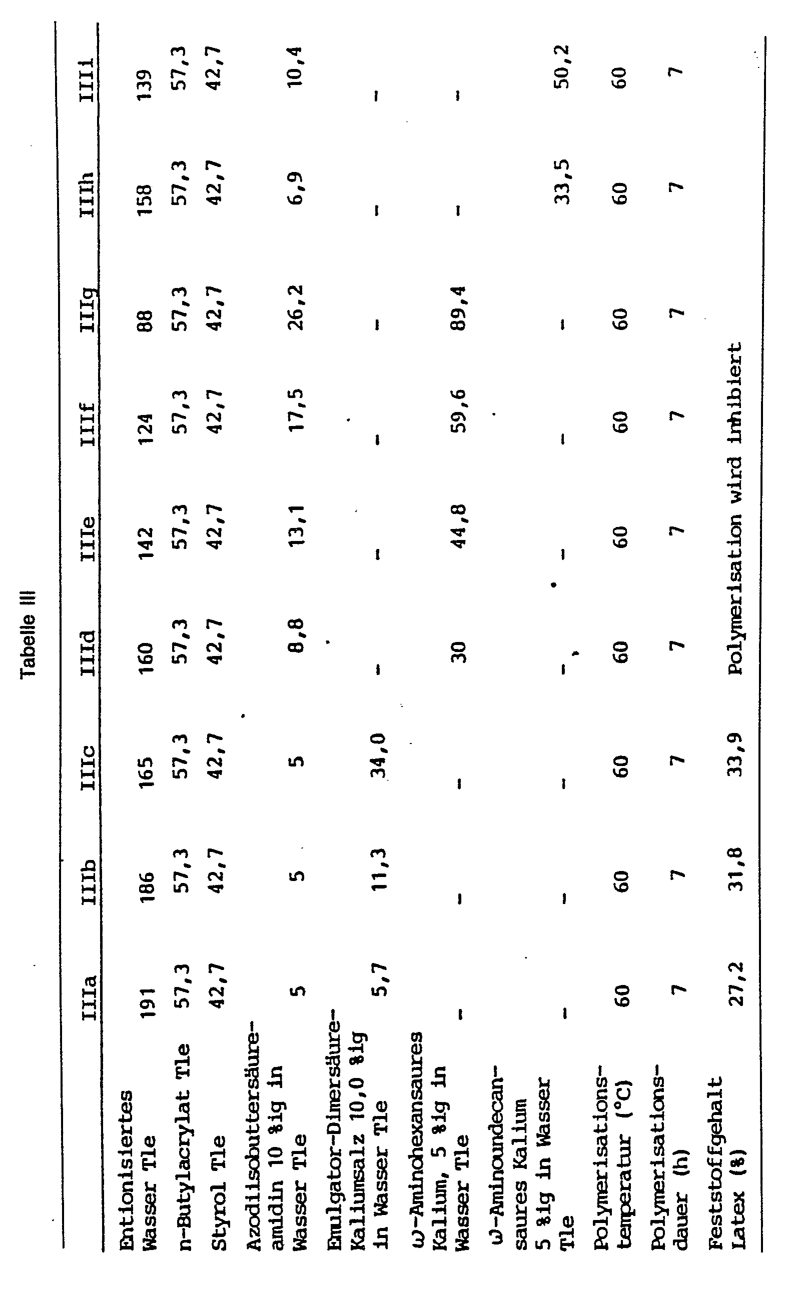

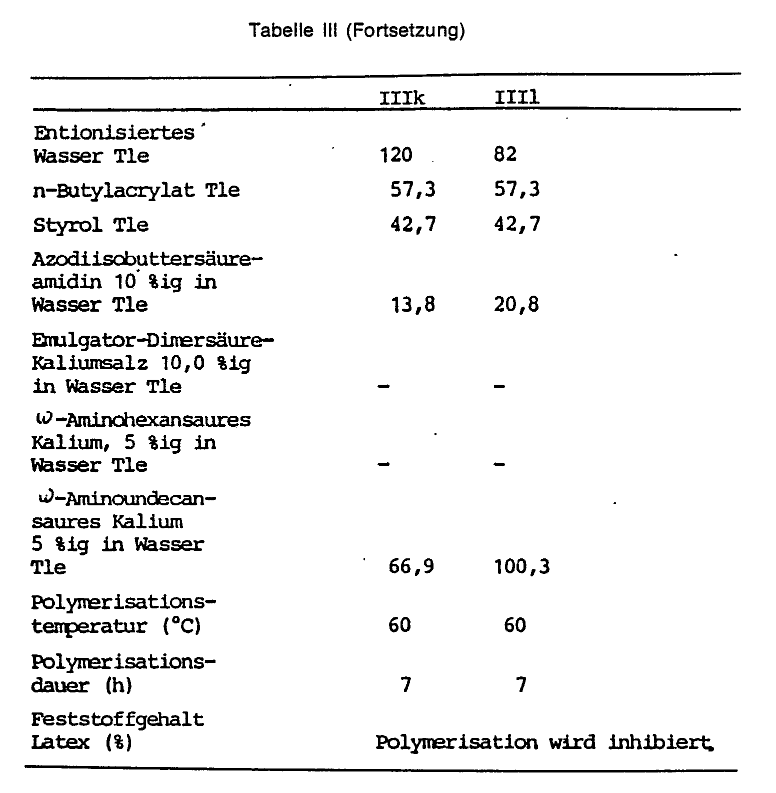

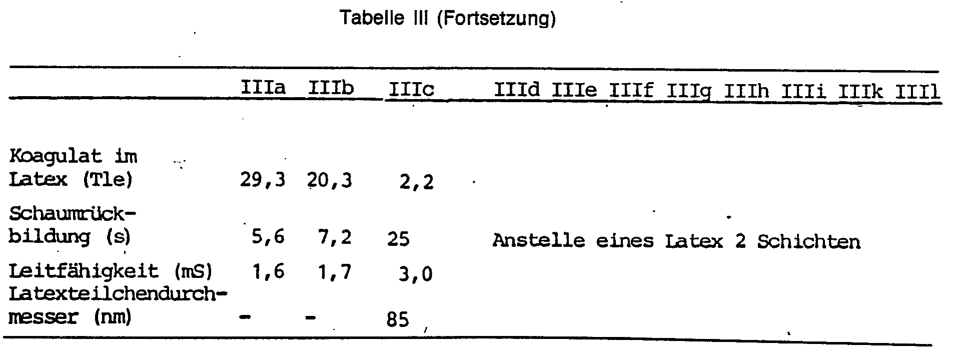

Wie aus Tabelle III ersichtlich, entstehen im Falle von Dimersäure-Dikaliumsalz als Emulgator bei niederer Dosierung Latices mit hohem Koagulatanteil. Die elektrische Leitfähigkeit dieser Latices liegt mit 1,6 und 1,7 mS ähnlich wie jene der Latices If und Ilf (gemäß Erfindung) der Tabelle I und II und ihre Tendenz zur Schaumbildung ist ebenfalls gering ; sie besitzen jedoch einen um den Faktor von ca. 10 höheren Koagulatanteil.

Wird nun der Anteil an Dimersäure-Emulgator so weit gesteigert, daß die Koagulatmenge etwa um den Faktor 10 zurückgeht (vgl. Versuch Illc in Tabelle III) so wird die elektrische Leitfähigkeit der Dispersion verdoppelt und die Tendenz zur Schaumbildung erhöht. Demgegenüber weisen die Dispersionen If in Tabelle I und Ilf in Tabelle II eine niedrigere elektrische Leitfähigkeit bei vergleichbarer Tendenz zur Schaumbildung sowie vergleichbarer Koagulatbildung auf. Werden die Dispersionen If sowie Ilf thermisch nachbehandelt, so kann ein weiterer Rückgang der elektrischen Leitfähigkeit auf Werte von etwa 1,0 bis 1,3 mS beobachtet werden.

Bei Verwendung der Kaliumsalze der w-Aminohexansäure bzw. der w-Aminoundecansäure als Emulgator und Azodiisobuttersäureamidin als Initiator (siehre Tabelle III, Versuch Illd-Ille) wird die Polymerisation des n-Butyl-acrylat-Styrol-Gemisches inhibiert. Dies ist auch noch dann der Fall, wenn die Initiatormenge drastisch erhöht wird. Ähnliche Ergebnisse erhält man auch, wenn anstelle des Azodiisobuttersäureamids als Initiator Azodi-iso-buttersäure-iminoethylether verwendet wird. Demgegenüber werden mit den erfindungsgemäßen Azoemulgatoren Latices mit wenig Koagulat, geringer Schaumbildungstendenz und geringer Leitfähigkeit erhalten.

### Vergleichsversuche (Tabelle IV)

Die Versuche werden analog Beispiel 2 durchgeführt. Als « Azoemulgatoren » werden
a) α,α'-Azo-(α-methyl-y-sulfo)-buttersäuredinitril der Formel 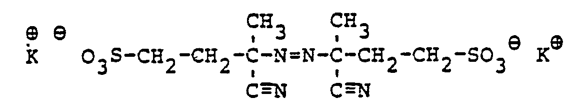 (vgl. DE-AS 1 111 395 = US-PS 3 161 630) bzw.
b) α,α'-Azo-(α-methyl-y-carboxy)-buttersäuredinitril der Formel 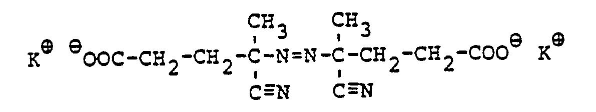 (vgl. US-PS 2 520 338) eingesetzt.

Einzelheiten und Ergebnisse sind aus Tabelle IV ersichtlich.

In keinem Fall wurde ein Latex erhalten ; die Reaktionsgemische waren schwer filtrierbare breiartige und koagulathaltige Massen.

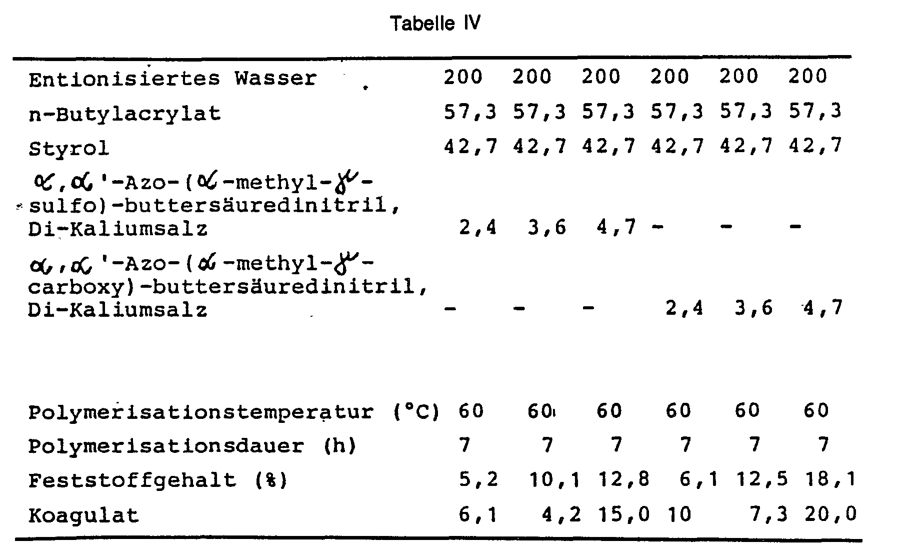

### Beispiel 4 (erfindungsgemäß)

### Polybutadienlatex mit Azoemulgator 2 gemäß Erfindung

In einen 6-Liter-Autoklav werden unter Stickstoffatmosphäre eingefüllt :

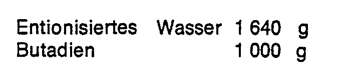

Das Gemisch wird auf 60 °C aufgeheizt. Danach werden 360 ml einer 10%igen Lösung des Azoemulgators A (gemäß Beispiel 1) aus einem kleinen Druckbehälter in den 6 Liter Autoklaven eingedrückt.

Nach einer Polymerisationszeit von 6 Stunden ist der Druck von 11,8 bar auf 3,4 bar abgefallen (bei 60 °C). Der Feststoffgehalt während der Polymerisation gezogener Proben beträgt nach Zugabe des Azoemulgators nach der

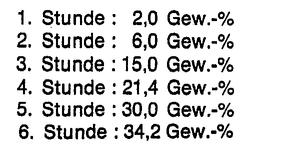

Der Latex besitzt einen Latexteilchendurchmesser von ca. 90 nm. Das Polymerisat ist nicht vollständig löslich in Toluol. Der lösliche Anteil besitzt eine Viskositätszahl von 0,8 (dl/g) in Toluol bei 25 °C. Koagulatanteil ca. 0,5 %, bezogen auf eingesetztes Butadien. Die elektrische Leitfähigkeit des Latex beträgt 1,5 mS, die Auslaufzeit im Auslaufbecher nach DIN 53 211 (4 mm Düse) ca. 24 Sekunden.

### Beispiel 5 (erfindungsgemäß, Azoemulgator 3 und Verwendung)

10,38 g p-Aminobenzolsulfonsäure (0,06 Mol)
3,37 g festes Kaliumhydroxid (0,06 Mol)
   werden in möglichst wenig Wasser (ca. 30 ml) gelöst. Die konzentriert wäßrige Lösung wird bei 25 °C zu einer Lösung von
16,9 g (0,03 Mol) der Verbindung : 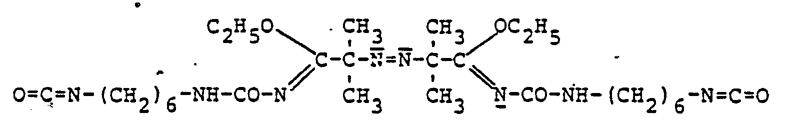 in 300 ml Tetrahydrofuran zugetropft und 5 Stunden nachgerührt.

Nach Absaugen des Reaktionsgemisches, wobei ca. 4 g wasserunlöslicher Rückstand verbleiben, wird das THF - Filtrat am Rotationsverdampfer im Vakuum schonend eingeengt (ca. 30 °C).

Der THF-freie Rückstand ist fest und wird in so viel Wasser aufgenommen, daß eine ca. 4,5 %ige Lösung entsteht. Nach Abfiltrieren geringfügiger ungelöster Anteile wird das trübe und schäumende Azoemulgator-Filtrat direkt als Polymerisationshilfsmittel verwendet (= Azoemulgator 3).

Im 6-Liter-Autoklav wird folgender Ansatz gemacht :
1 430 Tle. Wasser
573 Tle. Acrylsäure-n-butylester
427 Tle. Styrol
650 Tle. einer 4,5 %igen Lösung des obigen Azoemulgators 3.

Das Gemisch wird unter Rühren und unter Stickstoffatmosphäre auf 70 °C erhitzt und 7 Stunden bei dieser Temperatur gehalten. Danach wird auf Zimmertemperatur abgekühlt und der Latex abgelassen.

Es resultiert ein koagulatfreier Latex mit einem Feststoffgehalt von ca. 33 % (vollständiger Monomerenumsatz), einer elektrischen Leitfähigkeit von 1,5 mS, einer Latexteilchengröße von 140 nm im Durchmesser und einer geringen Tendenz zur Schaumbildung (nach kräftigem Schütteln Schaumrückgang nach 17 s).

## Patentansprüche

1. Oberflächenaktive Azoverbindungen der allgemeinen Formel (A) 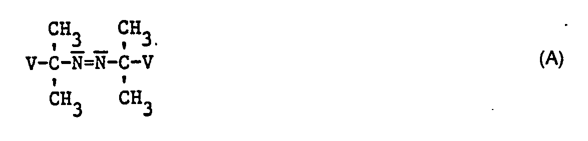 worin V die Bedeutung 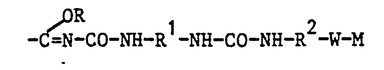 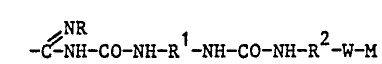 oder 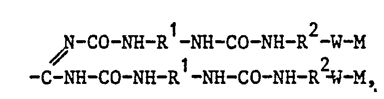 hat, wobei
R Alkyl mit 1-4 C-Atomen,
R¹ den um die Isocyanatgruppen verminderten Rest eines aliphatischen, aromatischen oder cycloaliphatischen Diisocyanats,
R² Alkylen mit 5-12 C-Atomen, Phenylen
W ―COO⁽⁻⁾. ―S0₃⁽⁻⁾ und
M Na⁽⁺⁾, K⁽⁺⁾, NH₄⁽⁺⁾ bedeuten.

2. Oberflächenaktive Azoverbindungen gemäß Anspruch 1, dadurch gekennzeichnet, daß
V die Bedeutung 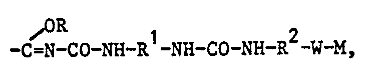 hat, wobei
R C₂H₅
R¹ den um die Isocyanatgruppen verminderten Rest: des 2,4- oder 2,6-toluylendüsocyanats bzw. von deren Mischungen ; des 4,4'-DÜsocyanatodiphenylmethans,. des Hexamethylendisocyanats, des 2,4,4-Trimethyl-1,6-diisocyanathohexans, des 1,12-Dodecamethylendiisocyanats, des Isophoron-diisocyanats,
R² Alkylen mit 5-10 C-Atomen, Phenylen
W ―C00⁽⁻⁾, -S0₃^{e} und
M Na⁽⁺⁾ oder K⁽⁺⁾ bedeuten.

3. Verwendung der oberflächenaktiven Azoverbindungen gemäß Anspruch 1 bei der Emulsionspolymerisation eines oder mehrerer olefinisch ungesättigter Monomerer als Initiator und Emulgator.

## Claims

1. Surface-active azo compounds of the general formula (A) 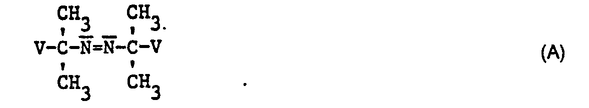 wherein
V has the meaning 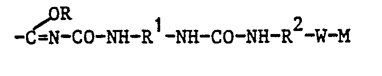 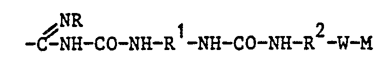 or 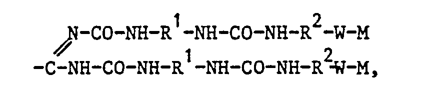 wherein
R denotes alkyl with 1-4 C atoms,
R¹ denotes the radical, without the isocyanate groups, of an aliphatic, aromatic or cycloaliphatic diisocyanate,
R² denotes alkylene with 5-12 C atoms, or phenylene,
W denotes ―C00⁽⁻⁾ or ―SO₃⁽⁻⁾ and
M denotes Na⁽⁺⁾, K⁽⁺⁾ or NH₄⁽⁺⁾.

2. Surface-active azo compounds according to Claim 1, characterised in that
V has the meaning 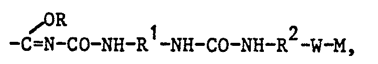 wherein
R denotes C₂H₅,
R¹ denotes the radical, without the isocyanate groups : of 2,4- or 2,6-tolylene diisocyanate or mixtures thereof ; of 4,4'-diisocyanatodiphenyl methane, of hexamethylene diisocyanate, of 2,4,4-trimethyl-1,6-diisocyanatohexane, of 1,12-dodecamethylene diisocyanate or of isophorone diisocyanate,
R² denotes alkylene with 5-10 C atoms, or phenylene,
W denotes ―COO⁽⁻⁾ or -So₃⊖ and
M denotes Na⁽⁺⁾ or K⁽⁺⁾.

3. Use of the surface-active azo compounds according to Claim 1 in the emulsion polymerisation of one or more olefinically unsaturated monomers as an initiator and emulsifier.

## Revendications

1. Composés azoïques tensioactifs, de formule générale (A) 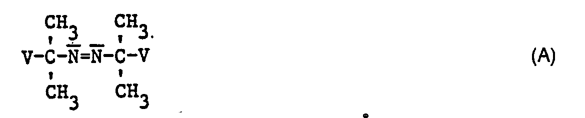 dans laquelle V a pour signification 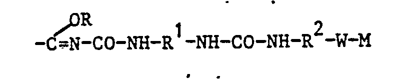 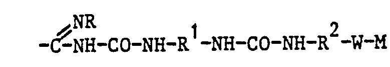 ou bien 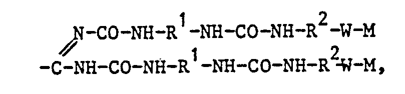 où
R représente un radical alkyle ayant 1 à 4 atomes de carbone,
R¹ représente le radical, sans les groupes isocyanate, d'un diisocyanate aliphatique, aromatique ou cycloaliphatique,
R² représente un radical alkylène ayant 5 à 12 atomes de carbone, phénylène,
W représente ―COO⁽⁻⁾, -SO₃⁽⁻⁾ et
M représente Na⁽⁺⁾, K⁽⁺⁾, NH₄⁽⁺⁾.

2. Composés azoïques tensioactifs selon la revendication 1, caractérisés en ce que V a pour signification 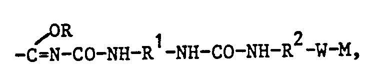 où
R représente C₂H_{S},
R¹ représente le radical, diminué du groupe isocyanate, du 2,4- ou 2,6-diisocyanate de toluylène ou de leurs mélanges ; du 4,4'-diisocyanatodiphénylméthane. du diisocyanate d'hexaméthylène, du 2,4,4-triméthyl-1,6-diisocyanatohexane, du 1,12-diisocyanate de dodécaméthylène, du diisocyanate d'isophorone,
R² représente un radical alkylène ayant 5 à 10 atomes de carbone, un radical phénylène,
W représente ―COO⁽⁻⁾, S0₃⁽⁻⁾ et
M représente Na⁽⁺⁾ ou K⁽⁺⁾.

3. Utilisation des composés azoïques tensioactifs selon la revendication 1, comme amorceurs et émulsifiants, dans la polymérisation en émulsion d'un ou plusieurs monomères à insaturation oléfinique.
